## Europäisches Patentamt

## European Patent Office

## Office européen des brevets

(19)

(11) Veröffentlichungsnummer: **0 019 046 B1**

(12) # EUROPÄISCHE PATENTSCHRIFT

(45) Veröffentlichungstag der Patentschrift:
27.07.83

(51) Int. Cl.³: **C 07 D 307/60**

(21) Anmeldenummer: **80101244.4**

(22) Anmeldetag: **11.03.80**

(54) **Verfahren zur kontinuierlichen Abscheidung von Maleinsäureanhydrid aus Prozessgasen.**

(30) Priorität: **21.03.79 IT 4843679**

(43) Veröffentlichungstag der Anmeldung:
**26.11.80 Patentblatt 80/24**

(45) Bekanntmachung des Hinweises auf die Patenterteilung:
**27.07.83 Patentblatt 83/30**

(84) Benannte Vertragsstaaten:
**AT BE CH DE FR GB LU NL SE**

(56) Entgegenhaltungen:
**CH-A-170 081**
**FR-A-1 386 278**
**FR-A-2 163 495**

(73) Patentinhaber: **ALUSUISSE ITALIA S.p.A., Via Vittor Pisani, 31, I-20124 Milano (IT)**

(72) Erfinder: **Neri, Amleto, Via Cifrondi 14, Bergamo (IT)**
Erfinder: **Sanchioni, Sergio, Via Caldara 2, Bergamo (IT)**

(74) Vertreter: **von Füner, Alexander, Dr. et al, Patentanwälte v. Füner, Ebbinghaus, Finck Mariahilfplatz 2 & 3, D-8000 München 90 (DE)**

## Verfahren zur kontinuierlichen Abscheidung von Maleinsäureanhydrid aus Prozeßgasen

Es ist bekannt, Maleinsäureanhydrid durch katalytische Oxydation in der Dampfphase von Benzol, gelegentlich auch n-Butan, Buten und anderen C₄-Fraktionen, herzustellen. Nach bekannten Methoden wird das Maleinsäureanhydrid auf die nachfolgend beschriebene Weise aus den Prozeßgasen gewonnen.

Zuerst werden die Gase auf Temperaturen von 55 bis 60° C gekühlt. Diese Temperatur liegt unter dem sogenannten Taupunkt von Maleinsäureanhydrid, aber noch über dem Taupunkt von Wasser, das seinerseits auch ein Beiprodukt der Reaktion darstellt. So können rund 50 bis 60% des Maleinsäureanhydrids aus den Prozeßgasen kondensiert werden. Je tiefer die Kondensationstemperatur gewählt wird, um so mehr wird das im Gas enthaltene Wasser durch das kondensierende Maleinsäureanhydrid, unter Bildung von Maleinsäure, gebunden. Die Bildung von Maleinsäure ist aber unerwünscht, da diese ein festes Produkt darstellt und in geschmolzenem Maleinsäureanhydrid schlecht löslich ist. Das führt zu Verstopfungen in den Apparaturen, woraus Durchsatz- und Ausbeuteverluste resultieren.

Das nichtkondensierte Maleinsäureanhydrid wird durch Waschen des Gasstromes mit Wasser als Maleinsäure gewonnen.

Die Maleinsäure muß dann wieder durch eine Dehydrierung in das Maleinsäureanhydrid umgewandelt werden. Durch Isomerisierungsprozesse kann sich dann auch Fumarsäure bilden, was letztlich zu großen Ausbeuteverlusten führt.

Im US-Patent 3 891 680 wird beschrieben, die Waschoperation in Estern aus Phthalsäure mit C₄ − C₈-Alkoholen durchzuführen. Gemäß deutscher Offenlegungsschrift 2 444 824 wird Dibenzylbenzol als Waschflüssigkeit eingesetzt. Schließlich beschreibt die englische Patentschrift 1 443 411 Polymethylbenzophenone als Waschflüssigkeit. Diese Systeme weisen aber gravierende Mängel auf, wie mangelnde Stabilität der Waschflüssigkeit, insbesondere der Phthalsäureester, was zu zusätzlichen Aufwendungen für den Ersatz der verlorenen Waschflüssigkeit führt, die hohen Kosten für die Waschflüssigkeit im Falle der Polymethylbenzophenone und schließlich, daß sowohl die Polymethylbenzophenone als auch die Dibenzylbenzole bei Raumtemperatur fest sind.

Aufgabe der Erfindung ist es, Waschflüssigkeiten respektive Lösungsmittel für das Maleinsäureanhydrid vorzuschlagen, welche die genannten Nachteile nicht aufweisen.

Erfindungsgemäß wurde das dadurch erreicht, daß man als Lösungsmittel die Dialkylester mit 4 bis 8 C-Atomen in jeder Alkylgruppe der Hexahydrophthalsäure, Tetrahydrophthalsäure, Methyltetrahydrophthalsäure oder Methylhexahydrophthalsäure anwendet.

Die Anwendung der erfindungsgemäßen Lösungsmittel ist besonders vorteilhaft, wenn als Prozeßgas die Reaktionsgase aus der katalytischen Oxydation von n-Butan in der Dampfphase angewendet werden. In diesem Fall ist die Wasserkonzentration in den Prozeßgasen viel größer als beispielsweise bei einem Verfahren, ausgehend von Benzol. So wäre es bei der Oxydation von n-Butan, bedingt durch den großen Wassergehalt, nur möglich, 30 bis 35% Maleinsäureanhydrid durch partielle Kondensation abzutrennen. Die verbleibenden 65 bis 70% müssen mit Wasser als Lösungsmittel ausgewaschen werden und würden als Maleinsäure anfallen. Unter großem Energieaufwand und Ausbeuteverlusten müßte die Maleinsäure vom Wasser getrennt und zum Maleinsäureanhydrid dehydriert werden.

Das erfindungsgemäße Verfahren wird in an sich bekannten Vorrichtunten ausgeführt. Die Prozeßgase aus der Reaktion können zuerst einer partiellen Kondensation unterworfen werden. Partiell kondensierte oder nichtkondensierte Prozeßgase werden in wenigstens eine Waschkolonne eingeführt. Das kann beispielsweise eine Bodenkolonne oder eine mit Raschigringen gefüllte Kolonne sein.

Das Prozeßgas wird in eine erste Kolonne eingeführt. Dem Gasstrom fließt das Lösungsmittel entgegen und belädt sich zunehmend mit dem Maleinsäureanhydrid. Die Temperatur und die Maleinsäureanhydridkonzentration des Gasstromes sinkt dabei, während sich das Lösungsmittel immer mehr mit Maleinsäureanhydrid anreichert. In entsprechender Weise können sich an die erste Kolonne weitere Kolonnen anschließen. Die Grenze der Adsorption des Maleinsäureanhydrids ist durch den Taupunkt des Wassers in den Reaktionsgasen gesetzt.

Es ist möglich, bis über 98% des Maleinsäureanhydrids ohne nennenswerte Maleinsäurebildung aus den Prozeßgasen herauszuwaschen.

Kontinuierlich oder allenfalls stufenweise kann das Gemisch, enthaltend 10 bis 30% Maleinsäureanhydrid, im Lösungsmittel einer fraktionierten Destillation zugeführt werden. Das Maleinsäureanhydrid wird abdestilliert und die hochsiedenden Lösungsmittel können im Kreislauf wieder der Adsorptionskolonne zugeführt werden.

Die erfindungsgemäßen Lösungsmittel weisen eine Reihe von bemerkenswerten Vorteilen auf. Sie sind flüssig bei Raumtemperatur und ihre Viskosität ist relativ niedrig. Beispielsweise sind die bekannten Polymethylbenzophenone oder die Dibenzylbenzole bei Raumtemperatur fest. Die erfindungsgemäßen Lösungsmittel haben hohe Siedepunkte. Bei den Adsorptionstemperaturen für das Maleinsäureanhydrid aus den Prozeßgasen weisen die Lösungsmittel deshalb nur niedrige Dampfdrücke auf. Durch die Gassättigung gehen nur vernachlässigbar kleine Mengen an Lösungsmittel verloren.

Die erfindungsgemäßen Lösungsmittel weisen auch eine hohe chemische Stabilität auf. Sie lassen sich nach der Adsorption wieder vom Maleinsäureanhydrid durch Destillation trennen und wieder in den Adsorptionsprozeß zurückführen. Mit dem Phthalsäureester, beispielsweise bekannt aus der US-PS 3 891 680 treten stets Verluste auf, die ergänzt werden müssen.

Schließlich ist die Affinität der erfindungsgemäßen Lösungsmittel zu Wasser vernachlässigbar klein. Die Bildung von Maleinsäure reduziert sich daher auf ein unbedeutendes Maß.

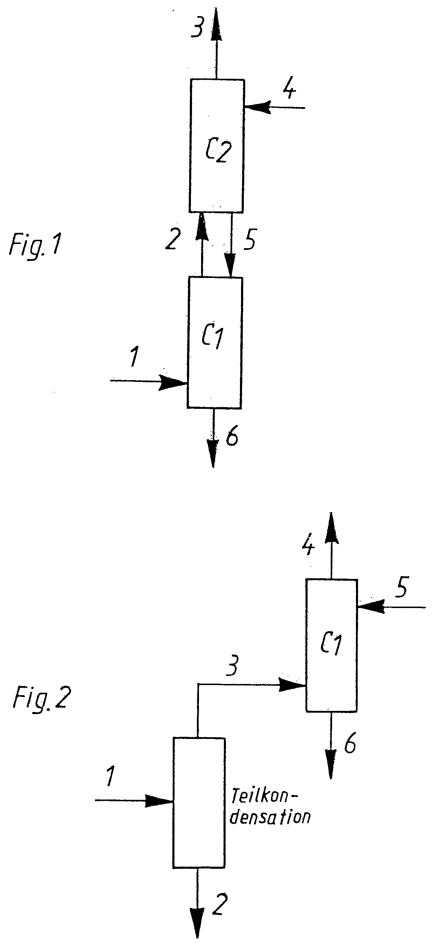

Fig. 1 und 2 zeigen jeweils ein Fließschema des erfindungsgemäßen Verfahrens.

Das erfindungsgemäße Verfahren verläuft wie folgt:

Die zu reinigenden Abgase, die Maleinsäureanhydrid und Wasser enthalten, werden, wie in Fig. 1 gezeigt, in ein aus zwei Kolonnen C1, C2 (bestehendes Absorptionssystem) über Leitung 1 geleitet. Das Abgas tritt in die erste Absorptionskolonne C1 ein und wird dort im Gegenstrom mit dem Lösungsmittel gewaschen. Die über Leitung 2 austretenden Gase gelangen dann in die Absorptionskolonne C2, wo sie noch einmal mit dem Lösungsmittel gewaschen werden. Über Leitung 4 wird Maleinsäureanhydrid enthaltendes Lösungsmittel zugegeben und über Leitung 5 an Maleinsäureanhydrid angereichertes Lösungsmittel abgezogen und nach Durchgang durch einen Wärmeaustauscher in den Kopf von Kolonne C1 eingeleitet. Am Boden der Kolonne C1 wird das an Maleinsäureanhydrid reiche Maleinsäureanhydrid-Lösungsmittel abgezogen. Über Kopf der Kolonne C2 gehen über die Leitung 3 die von Maleinsäureanhydrid gereinigten Gase ab.

Die bei 6 abgezogene Maleinsäureanhydridlösung wird dann anschließend fraktioniert destilliert, wobei man reines Maleinsäureanhydrid und die maleinsäureanhydridhaltige Lösung erhält, die bei 4 wieder der Kolonne C2 zugespeist wird.

Nach Fig. 2 gelangen die zu reinigenden Abgase über 1 in ein Teilkondensationssystem, wo der gekühlte Teil durch Leitung 2 am Boden abgezogen wird. Die noch Maleinsäureanhydrid enthaltenden nichtkondensierten Gase werden über die Leitung 3 in die Absorptionskolonne C1 weitergeleitet und dort im Gegenstrom mit dem Lösungsmittel gewaschen. Die gewaschenen Gase treten über die Leitung 4 aus. Über die Leitung 5 wird das Maleinsäureanhydrid enthaltende Lösungsmittel eingespeist und über Leitung 6 das an Maleinsäureanhydrid reiche Gemisch abgezogen.

## Beispiel 1

100 kg Abgase der katalytischen Oxydation von n-Butan zu Maleinsäureanhydrid, enthaltend 2,65 kg Maleinsäureanhydrid und 4,8 kg Wasser, leitet man in ein aus zwei Kolonnen bestehendes Absorptionssystem ein (s. schematisches Fließbild Fig. 1).

Die Gase treten durch Leitung 1 in die erste Absorptionskolonne C1 bei einer Temperatur von 80°C ein. In Kolonne C1 werden die Gase im Gegenstromverfahren bei 80° mit Di-butylhexahydrophthalat gewaschen.

Die aus Kolonne C1 austretenden Gase werden auf 60°C gekühlt (mit Hilfe eines in Fig. 1 nicht dargestellten Wärmeaustauschers) und über Leitung 2 in die Absorptionskolonne C2 (60°C) eingeleitet.

In Kolonne C2 werden die Gase noch einmal mit Di-butyl-hexahydrophthalat bis zu einem Gehalt an Maleinsäureanhydrid von nur 0,06 kg beim Austreten aus Kolonne C2 (über Leitung 3) gewaschen.

Die Gesamtmenge an absorbiertem Maleinsäureanhydrid beträgt bei einer Ausbeute von 97,7% 2,59 kg.

16,53 kg 1,1 Gew.-% Maleinsäureanhydrid enthaltendes Lösungsmittel leitet man in den Kopf von Kolonne C2 (über Leitung 4). Am Ausgang von Kolonne C2 treten 17,11 kg Maleinsäureanhydrid-Di-butyl-hexahydrophthalat-Lösung, enthaltend 4,4% Maleinsäureanhydrid, über Leitung 5 aus. Diese Lösung tritt nach Erwärmen durch einen in Fig. 1 nicht dargestellten Wärmeaustauscher auf 80°C in den Kopf von Kolonne C1 ein.

Am Ausgang von Kolonne C1 (über Leitung 6) erhält man 19,12 kg einer 14,5 Gew.-% Maleinsäureanhydrid enthaltenden Maleinsäureanhydrid-Di-butyl-hexahydrophthalat-Lösung.

In Kolonne C1 findet eine Absorption von Maleinsäureanhydrid bis zu 2,01 kg (77,6% des absorbierten Maleinsäureanhydrids), in Kolonne C2 eine Absorption von Maleinsäureanhydrid bis zu 0,58 kg (22,4% des absorbierten Maleinsäureanhydrids) statt.

Die Gesamtanzahl von Absorptionsstufen (theoretisch) beträgt 6 (davon 3 in Kolonne C1 und die restlichen 3 Stufen in Kolonne C2).

Die 14,5%ige Maleinsäureanhydrid-Lösung leitet man in fraktioniertes Destillationssystem (nicht dargestellt in Fig. 1), in dem man praktisch reines Maleinsäureanhydrid als Vorlaufprodukt (2,59 kg) und 1,1 Gew.-% Maleinsäureanhydrid enthaltendes Di-butyl-hexahydrophthalat als Nachlaufprodukt erhält. Das erhaltene Di-butyl-hexahydrophthalat wird in das Maleinsäureanhydrid-Absorptionssystem über Leitung 4 zurückgeleitet.

## Beispiel 2

101,54 kg Abgase (s. Fig. 2), enthaltend 2,96 kg Maleinsäureanhydrid und 2,82 kg Wasser der katalytischen Oxydation von Benzol zu Maleinsäureanhydrid (Leitung 1), werden in ein Teilkondensationssystem eingeleitet, in dem durch Kühlung des Gasgemisches auf 58°C 1,54 kg Maleinsäureanhydrid kondensiert werden (Leitung 2). Das nichtkondensierte Gas (100 kg) enthält 1,42 kg Maleinsäureanhydrid, wird über Leitung 3 in die Absorptionskolonne C1 weitergeleitet und bei 58°C im Gegenstromverfahren mit Di-isobutyl-tetrahydrophthalat gewaschen.

Die aus der Kolonne austretenden Gase (Leitung 4) enthalten nur 0,07 kg Maleinsäureanhydrid; die restlichen 1,35 kg an Maleinsäureanhydrid sind im Di-isobutyl-tetrahydrophthalat absorbiert.

Die Absorptionsausbeute beträgt 95,1%. Aus diesem Grunde beträgt unter Berücksichtigung dessen, daß 1,54 kg Maleinsäureanhydrid direkt durch Teilkondensation erhalten wurden, die Gesamtausbeute an Maleinsäureanhydrid 97,6%.

In Kolonne C1 finden vier theoretische Absorptionsstufen statt. 8,25 kg Maleinsäureanhydrid-Di-isobutyl-tetrahydrophthalat-Lösung, enthaltend 1,1 Gew.-% Maleinsäureanhydrid, werden in den Kopf der Kolonne (über Leitung 5) eingeleitet.

Die Menge an mit Maleinsäureanhydrid angereicherter 15 Gew.-% davon enthaltender Lösung beträgt 9,60 kg. Sie wird durch fraktionierte Destillation (nicht dargestellt in Fig. 2) getrennt. Das erhaltene Produkt besteht aus 1,35 kg fast reinem Maleinsäureanhydrid (Vorlauf) und 8,26 kg Maleinsäureanhydrid-di-isobutyl-tetrahydrophthalat (Nachlauf), enthaltend 1,1 Gew.-% Maleinsäureanhydrid.

Die zweite Lösung wird über Leitung 5 in Kolonne C1 zurückgeleitet, während das als Vorlaufprodukt fast reine Maleinsäureanhydrid dem durch Teilkondensation erhaltenen (Leitung 2) Maleinsäureanhydrid zugegeben und noch einmal zwecks Erhalt praktisch reinen Maleinsäureanhydrids teilkondensiert und nochmals destilliert wird.

Beispiel 3

100 kg Abgase der katalytischen Oxydation von isomeren n-Butenen zu Maleinsäureanhydrid, enthaltend 3,06 kg Maleinsäureanhydrid und 4,97 kg Wasser, leitet man in eine aus zwei Kolonnen (s. schematisches Fließbild Fig. 1) bestehendes Absorptionssystem ein.

Die Temperatur des in die erste Absorptionskolonne C1 über Leitung 1 eintretenden Gases beträgt 75°C. In Kolonne C1 wird das Gas im Gegenstromverfahren bei 75°C mit Di-isobutyl-hexahydrophthalat gewaschen.

Die aus Kolonne C1 austretenden Gase werden mit Hilfe eines in Fig. 1 nicht dargestellten Wärmeaustauschers auf 65°C gekühlt und über Leitung 2 in eine bei 65°C arbeitende Absorptionskolonne C2 eingeleitet.

In Kolonne C2 werden die Gase zuletzt mit Di-isobutyl-hexahydrophthalat derart gewaschen, daß sie beim Austreten aus dieser Kolonne nur 0,06 kg Maleinsäure enthalten.

Die Gesamtmenge an absorbiertem Maleinsäureanhydrid beträgt bei einer Absorptionsausbeute an 98% 3,00 kg. Die Menge an über Leitung 4 in den Kopf der Kolonne C2 eingeleitetem Lösungsmittel beträgt bei einem Gehalt an Maleinsäureanhydrid von 0,5 Gew.-% 28,42 kg.

Aus Kolonne C2 treten über Leitung 5 28,79 kg 1,77 Gew.-% Maleinsäureanhydrid enthaltende Maleinsäureanhydrid-Di-isobutyl-hexahydrophthalat-Lösung aus. Diese mit Hilfe eines in Fig. 1 nicht dargestellten Wärmeaustauschers vorerwärmte Lösung wird in den Kopf der Kolonne C1 eingeleitet.

Am Ausgang von Kolonne C1 (Leitung 6) erhält man 31,42 kg einer 10 Gew.-% Maleinsäureanhydrid enthaltenden Maleinsäureanhydrid-Di-isobutyl-hexahydrophthalat-Lösung.

In Kolonne C1 werden 2,63 kg Maleinsäureanhydrid (87,7% an absorbiertem Maleinsäureanhydrid) absorbiert; in Kolonne C2 beträgt die Menge an absorbiertem Maleinsäureanhydrid 0,37 kg (12,3% absorbiertes Maleinsäureanhydrid).

Es finden 4 theoretische Absorptionsstufen statt, davon 2 in Kolonne C1 und zwei in Kolonne C2.

Die 10% Maleinsäureanhydrid enthaltende Lösung wird in ein fraktioniertes Destillationssystem (nicht in Fig. 1 dargestellt) geleitet, worin man praktisch reines Maleinsäureanhydrid als Vorlaufprodukt (3,00 kg) und 0,5 Gew.-% Maleinsäureanhydrid enthaltendes Di-isobutyl-hexahydrophthalat als Nachlaufprodukt (3,00 kg) erhält.

Das erhaltene Di-isobutyl-hexahydrophthalat wird über Leitung 4 in das Maleinsäureanhydrid-Absorptionssystem zurückgeleitet.

Beispiel 4

100,74 kg (s. Fig. 2) Abgase der katalytischen Oxydation von n-Butan zu Maleinsäureanhydrid, enthaltend 2,66 kg Maleinsäureanhydrid und 4,82 kg Wasser, werden über Leitung 1 in ein bei 64°C arbeitendes Teilkondensationssystem geleitet. Auf diese Weise werden über Leitung 2 0,74 kg Maleinsäureanhydrid (ca. 28% des in der Reaktionsphase erhaltenen Maleinsäureanhydrids) wiedergewonnen.

100 kg nichtkondensierte Gase, enthaltend 1,92 kg Maleinsäureanhydrid, werden über Leitung 3 in die Absorptionskolonne C1 geleitet und darin bei 64°C im Gegenstromverfahren mit Di-hexylmethylhexahydrophthalat gewaschen.

Das aus Kolonne C1 über Leitung 4 austretende Gas enthält nur 0,05 kg Maleinsäureanhydrid. Die Ausbeute beträgt 97,4%. Unter Berücksichtigung dessen, daß bei der Teilkondensation (Leitung 2) 0,74 kg Maleinsäureanhydrid wiedergewonnen werden, beträgt die Gesamtausbeute an Maleinsäureanhydrid 98,1%.

In Kolonne C1 finden 3 theoretische Absorptionsstufen statt. 17,72 kg eines Gemisches aus Maleinsäureanhydrid und Di-hexylmethylhexahydrophthalat mit einem Gehalt von 0,5 Gew.-% an Maleinsäureanhydrid werden in den Kopf von Kolonne C1 (Leitung 5) geleitet.

Die an Maleinsäureanhydrid reiche (10% Maleinsäureanhydrid) aus dem Boden von Kolonne C1 über Leitung 6 austretende Lösung wird in ein in Fig. 2 nicht dargestelltes fraktioniertes Destillationssystem geleitet; als Vorlaufprodukt erhält man 1,87 kg fast reines Maleinsäureanhydrid und als Nachlaufprodukt 17,72 kg eines Gemisches aus Maleinsäureanhydrid und Di-hexylmethylhexahydrophthalat mit einem Gehalt an 0,5 Gew.-% Maleinsäureanhydrid.

Das zweite Gemisch wird in den Kopf von Kolonne C1 über Leitung 5 zurückgeleitet, während das durch fraktionierte Destillation als Vorlaufprodukt erhaltene Maleinsäureanhydrid mit dem durch Teilkondensation (Leitung 2) erhaltenen Maleinsäureanhydrid vereinigt und nochmals durch fraktionierte Destillation (nicht dargestellt in Fig. 2) zwecks Erhalt von praktisch reinem Maleinsäureanhydrid gereinigt wird.

## Patentansprüche

1. Verfahren zur kontinuierlichen Abscheidung von Maleinsäureanhydrid aus Prozeßgasen aus der katalytischen Oxydation von Kohlenwasserstoffen in der Dampfphase durch Behandlung der Prozeßgase mit einem Lösungsmittel, dadurch gekennzeichnet, daß man als Lösungsmittel die Dialkylester mit 4 bis 8 C-Atomen in jeder Alkylgruppe der Hexahydrophthalsäure, Tetrahydrophthalsäure, Methyltetrahydrophthalsäure oder Methylhexahydrophthalsäure anwendet.

2. Verfahren nach Anspruch 1, dadurch gekennzeichnet, daß man als Prozeßgase die Reaktionsgase aus der katalytischen Oxydation von n-Butan in der Dampfphase anwendet.

## Claims

1. Process for the continuous separation of maleic anhydride from process gases from the catalytic oxidation of hydrocarbons in the vapour phase by treatment of the process gases with a solvent, characterised in that, as solvent, one uses the dialkyl esters, with 4 to 8 C-atoms in each alkyl group, of hexahydrophthalic acid, tetrahydrophthalic acid, methyltetrahydrophthalic acid or methylhexahydrophthalic acid.

2. Process according to Claim 1, characterised in that, as process gases, one uses the reaction gases from the Catalytic oxidation of n-butane in the vapour phase.

## Revendications

1. Procédé pour séparer en continu l'anhydride maléique à partir de gaz opératoires de l'oxydation catalytique d'hydrocarbures en phase vapeur par traitement des gaz opératoires à l'aide d'un solvant, caractérisé en ce que l'on utilise en tant que solvants les esters dialkyliques, contenant de 4 à 8 atomes de carbone dans chaque groupe alkyle, de l'acide hexahydrophtalique, de l'acide tétrahydrophtalique, de l'acide méthyltétrahydrophtalique ou de l'acide méthylhexahydrophtalique.

2. Procédé selon la revendication 1, caractérisé en ce que les gaz opératoires traités sont les gaz de réaction de l'oxydation catalytique du n-butane en phase vapeur.

Fig.1

Fig.2

Teilkondensation